# EUROPÄISCHE PATENTSCHRIFT

(11) **EP 1 455 662 B1**
(45) Veröffentlichungstag und Bekanntmachung des Hinweises auf die Patenterteilung: **02.12.2009**
(21) Anmeldenummer: 02803017.9
(22) Anmeldetag: 13.11.2002
(51) Int. Cl.: A61B 17/17

(54) **ZIELGERÄT FÜR EINEN FRAKTURNAGEL**
TARGETING DEVICE FOR A FRACTURE PIN
DISPOSITIF DE VISEE DESTINE A UNE BROCHE A FRACTURE

(30) Priorität: 13.11.2001 DE 10155495; 14.11.2001 DE 10155743
(43) Veröffentlichungstag der Anmeldung: 15.09.2004
(73) Patentinhaber: Tantum AG, 24537 Neumünster (DE)
(72) Erfinder: JENSEN, Harm-Iven, 24214 Noer (DE)
(74) Vertreter: Wenzel & Kalkoff
(86) Internationale Anmeldenummer: PCT/EP2002/012696
(87) Internationale Veröffentlichungsnummer: WO 2003/041595

(56) Entgegenhaltungen:
- CH-A- 668 692
- DE-A- 4 238 582
- DE-U- 9 102 018
- DE-U- 20 002 988
- US-A- 5 776 194

## Beschreibung

Die Erfindung betrifft ein Zielgerät für einen Frakturnagel, umfassend einen Zielarm, der an seinem einen Ende ein Verbindungsmittel zur lösbaren Verbindung mit dem Frakturnagel aufweist, wenigstens einen an dem anderen freien Ende des Zielarms angeordneten vetstellbaren Aufnahmeabschnitt mit Zielbohrung , die einer quer zur Nagel-Längsachse verlaufenden Frakturnägel-Querdurchbohrung zur Aufnahme eines Knochenstiftes zugeordnet ist, sowie ein Feststellmittelzum Feststellen des Aufnahmeabschnitts an dem freien Zielarmende. Mit einem solchen Zielgerät verbindbare Frakturnägel werden zur Versorgung von Frakturen, vor allem von Frakturen der großen Röhrenknochen wie Femur (Oberschenkelknochen), Tibia (Schienenbein) und Humerus (Oberarmknochen) verwendet und sind dementsprechend in verschiedenen, den Indikationen angepaßten Ausführungsformen bekannt. Knochenstifte sind meist als Knochennägel oder Knochenschrauben ausgebildet und dienen insbesondere dazu, den Frakturnagel im Knochen zu fixieren, zu verriegeln oder Knochenfragmente miteinander zu verbinden.

Typische Erfordernisse beim Einsatz von Frakturnägeln werden am Beispiel eines Femurnagels erläutert, der zur Versorgung per- und subtrochantärer Frakturen des Femurs verwendet wird und zum Beispiel aus EP 0 321 170 bekannt ist. In einfacher Ausführung umfaßt der Femurnagel einen Marknagel und eine Schenkelhalsschraube. Der Marknagel weist in seinem proximalen Bereich eine Querbohrung zur Aufnahme der Schenkelhalsschraube auf. Die Schenkelhalsschraube weist an ihrem proximalen Ende ein Außengewinde auf. Zur Implantation des Femurnagels wird in einem ersten Schritt der Marknagel mit seinem distalen Ende voran in den Markraum des Femurs eingeführt. Dabei muß der Marknagel derart ausgerichtet werden, daß die Querbohrung in der vom Chirurgen gewünschten Richtung zu dem Schenkelhals verläuft. In einem zweiten Schritt wird die Schenkelhalsschraube mit ihrem proximalen Ende voran von außen zunächst durch die Weichteilgewebe des Oberschenkels und weiter durch den Knochen bis zu der Querbohrung geschoben und in diese eingeführt, bis ihr proximales Ende auf der anderen Seite aus der Querbohrung austritt. In einem dritten Schritt schließlich wird die Schenkelhalsschraube durch den Schenkelhals bis in den Schenkelkopf hineingeschraubt. Nach dem ersten Schritt kann der Chirurg die Querbohrung nicht mehr sehen, da diese im Knochen liegt. Er muß aber deren Lage und Verlauf relativ zum Oberschenkel genau kennen, da beim zweiten Schritt zwangsläufig sowohl Weichteilgewebe als auch Knochen geschädigt werden und somit jeder Fehlversuch beim Einführen der Schenkelhalsschraube zusätzlichen Schaden bedeutet. Zwar kann man Lage und Verlauf mit Hilfe von Röntgengeräten sichtbar machen, jedoch ist Strahlenbelastung nach Möglichkeit zu vermeiden, und die Operationstechnik soll möglichst einfach sein.

Um Querdurchbohrungen des implantierten Frakturnagels aufzufinden, arbeitet eine Kategorie von Zielgeräten mit Röntgenstrahlen (z.B. DE 42 38 582 A, CH 668 692 A, US 5 776 194). Bei einer anderen Geräte-Kategorie, zu der das gattungsgemäße Zielgerät gehört, werden Zielgeräte fest mit einem Ende des Nagels verbunden, und ein bügelartiger Abschnitt weist mindestens eine Ziel-Durchbohrung auf, deren Achse mit der Achse einer Querdurchbohrung des Frakturnagels ausgerichtet ist, wenn letzterer am Zielgerät angebracht wird (z.B. EP 0 321 170 A1, US 6 126 661, US 5 334 192, DE 298 06 564 U1).

Aus der Gebrauchsmusterschrift DE 200 02 988 U1 ist ein gattungsgemäßes Zielgerät bekannt, das neben weiteren Aufnahmeabschnitten für den proximalen Bereich eines Frakturnagels mit einem verstellbaren Aufnahmeabschnitt am verlängerten freien Ende eines Zielarms versehen ist. Der Aufnahmeabschnitt muß in feste Position gesetzt und gehalten werden, um zugleich drei Bohrhülsen für drei zugeordnete Frakturnagel-Querdurchbohrungen auszurichten, und kann nur mit Rastmaß versetzt werden, um das Zielgerät für eine entsprechende Gruppe von drei distalen Querdurchbohrungen an unterschiedlich langen Frakturnägeln verwenden zu können. Ein Zielgerät mit bewegbaren Gruppen von Führungsbohrungen zur Anpassung an entsprechende Gruppen von Querdurchbohrungen an unterschiedlich langen Frakturnägeln ist auch aus der Gebrauchsmusterschrift DE 91 02 018 bekannt.

Es ist auch bekannt (DE 298 06 564 U1), einen Aufnahmeabschnitt lösbar zu befestigen, um ihn in Anpassung an den verwendeten Frakturnagel austauschen zu können. Zu diesem Zweck müssen unterschiedliche Aufnahmeabschnitte mit besonderer Kennung für zugehörige Frakturnägel bereitgehalten und verwendet werden. Bei einem weiteren bekannten Gerät wird ein Aufnahmeabschnitt mit lateralseitig dichter Folge und sogar sich überlappenden Öffnungen von Zielbohrungen vorgesehen (US 5 334 192). Die Verwendung eines solchen Zielgeräts bleibt eingeschränkt.

Danach bestehen Ziele der Erfindung darin, ein Zielgerät der genannten Kategorie zur verbesserten und universellen Verwendung für ein und denselben Frakturnagel und gegebenenfalls mehrere Frakturnägel zu schaffen.

Ziele der Erfindung werden in Verbindung mit den Merkmalen des eingangs genannten Zielgeräts dadurch erreicht, daß der Aufnahmeabschnitt durch einen verstellbaren Zielkopf gebildet wird, der zum Einstellen in unterschiedliche Zielpositionen für ein und denselben Frakturnagel stufig verstellbar ist, und daß das Feststellmittel durch ein Rastmittel zur stufigen Verstellung des Zielkopfes in wenigstens zwei vorgegebene Rastpositionen gebildet ist, wobei für ein und denselben Frakturnagel jede Rastposition eine Zielposition für eine zugeordnete Frakturnagel-Querdurchbohrung bestimmt. Der Zielkopf weist wenigstens eine Zielbohrung auf. Zum Ausrichten einer bzw. jeder Zielbohrung auf zugeordnete Frakturnagel-Querdurchbohrung ist der Zielkopf verstellbar an dem freien Ende des Zielarms angeordnet.. Mit der Erfindung ist insbesondere erreicht, daß durch einfache Positionsver- stellung ein und desselben Zielkopfes an dem freien Zielarmende unterschiedliche Bohrungspositionen ein und desselben Frakturnagels versorgt werden können. Infolge des jeweiligen Verstellweges lassen sich die zu verwendenden Zielbohrungen relativ einfach unterscheiden. Im Unterschied zu lediglich in austauschbare Aufnahmeabschnitte oder in einen Zielarm eingebrachten Zielbohrungen lassen sich erfindungsgemäß Zielbohrungsöffnungen mit lateral signifikanten Lochabständen vorsehen, wodurch die Operationstechnik verbessert wird. Im ganzen bildet der verstellbare Zielkopf des erfindungsgemäßen Zielgeräts ein veränderbares Mittel, das die Operationstechnik mit größter Zielpräzision unter Verwendung ein und desselben Zielgeräts verbessert. Das erfindungsgemäße Zielgerät ist für Frakturnägel im weiteren Sinne geeignet, nämlich für entsprechende derartige Implantate, bei deren Einsatz Gegenstände wie zum Beispiel eine Sonde, ein Bohrer, ein Knochenstift, ein Werkzeug, ein Bauteil od.dgl. Elemente von außerhalb des Körpers durch Gewebe hindurch mit großer Genauigkeit gehandhabt werden müssen.

In besonders bevorzugter und vorteilhafter Ausgestaltung sieht die Erfindung vor, daß der Zielkopf drehbar an dem freien Zielarmende angebracht ist. Besonders zweckmäßig kann der Zielkopf um eine Achse drehbar angeordnet werden, die sich in Verlängerung des freien Zielarmendes erstreckt. Die Drehachse kann man vorteilhaft so ausrichten, daß der Zielkopf um eine Achse drehbar ist, die in gemeinsamer Ebene mit der Längsachse eines mit Zielgerät verbundenen Frakturnagels zu liegen kommt. In bevorzugter Orientierung kann die Drehachse zumindest im wesentlichen parallel zur Längsachse eines mit dem Zielgerät verbundenen Frakturnagels liegen.

Nach einer weiteren Gestaltung der Erfindung ist es auch möglich, daß der Zielkopf drehbar um eine Achse angeordnet wird, die zumindest im wesentlichen rechtwinklig zu der Ebene liegt, die von der Längsachse eines mit dem Zielgerät verbundenen Frakturnagels und einer in Verlängerung des freien Zielarmendes sich erstreckenden Achse aufgespannt wird. Auch kann vorgesehen sein, daß der Zielkopf um eine Achse drehbar ist, die zumindest im wesentlichen rechtwinklig zu der Längsachse eines mit dem Zielgerät verbundenen Frakturnagels und in der Ebene liegt, die von der Längsachse des Frakturnagels und einer in Verlängerung des freien Zielarmendes sich erstreckenden Achse aufgespannt wird. Weiterhin kann in Kombination der genannten Freiheitsgrade vorgesehen sein, daß der Zielkopf an dem freien Zielarmende sphärisch, zweckmäßig mittels eines Kugelgelenkes gelagert wird. Insbesondere aufgrund der Drehverbindung und -verstellbarkeit des Zielkopfes können die Zielbohrungslöcher in bezug auf den mit jeder Drehverstellung sich ergebenden Lateralbereich des Zielkopfes in relativ großen signifikanten Abständen vorgesehen werden.

Eine weitere Ausgestaltung der Erfindung besteht darin, daß der Zielkopf verschiebbar, vorzugsweise in Richtung einer in Verlängerung des freien Zielarmendes sich erstrekkenden Achse, an dem freien Zielarmende angebracht wird. Die Schiebeverbindung kann in Verbindung mit einem Rastmittel zum Einstellen von Zielpositionen des Zielkopfes vorgesehen sein. Die Schiebeverbindung kann aber auch zur Schiebeverstellung des Zielkopfes ausgebildet sein, um ihn optional in unterschiedliche Zielpositionen zu bringen. Die Zielbohrungen werden dann durch Verschieben relativ zum Zielarmende und somit zum Frakturnagel und seinen Querbohrungen verstellt. Die Schiebeverbindung kann so gestaltet sein, daß ihre Schiebeachse, längs derer der Zielkopf schiebeverstellbar ist, zumindest im wesentlichen parallel zur Längsachse des Frakturnagels liegt. In Kombination damit oder separat können weitere Verstell-Schieberichtungen vorgesehen werden. Die Schiebeachse kann so orientiert werden, daß sie wenigstens im wesentlichen rechtwinklig zu der Ebene liegt, die von der Längsachse des Frakturnagels und der Längsachse des freien Zielarmendes aufgespannt wird. Auch kann sie so orientiert werden, daß sie zumindest im wesentlichen rechtwinklig zur Längsachse des Frakturnagels und zu der Ebene liegt, die von der Längsachse des Frakturnagels und einer in Richtung des freien Zielarmendes verlängerten Achse aufgespannt wird.

Das Zielgerät umfaßt ein Zielpositionen bestimmendes Feststellmittel, mit dem der Zielkopf in wenigstens zwei vorgegebenen Positionen feststellbar ist. Das Feststellmittel kann ein zwischen dem freien Zielarmende und dem Zielkopf ausgebildetes Spann- oder Klemmittel sein, mit dem Zielarm und Zielkopf in gegeneinander eingestellter Verstellposition gegen Verstellung gesichert werden. Zudem ist es denkbar , daß die Ziel-Verstellpositionen des Zielkopfes durch Markierungen der Positionen definiert und eingestellt werden. Vorgegebene, fakultativ einstellbare Zielpositionen werden dadurch eingerichtet , daß das Feststellmittel ein Rastmittel umfaßt. In Ausgestaltung der Erfindung mit drehverstellbar an dem freien Zielarmende angebrachtem Zielkopf umfaßt das Rastmittel Mittel zur stufigen Drehverstellung in vorgegebenen, Rastpositionen bestimmenden Winkelabständen. Vorteilhaft kann ein solches Rastmittel ein den Zielkopf gegen das freie Zielarmende vorspannendes Federmittel umfassen, mittels dessen der Zielkopf unter auf ihn gegen die Vorspannrichtung ausgeübter Zugkraft zwischen den Rastpositionen drehverstellbar ist.

Es können Mittel vorgesehen sein, um den Zielkopf insbesondere zum Reinigen von dem Zielarm abnehmen zu können. Mittels lösbarer Befestigung kann ein Zielkopf auch gegen einen anderen Zielkopf ausgewechselt werden, der zum Beispiel unterschiedlich angeordnete und/oder ausgebildete Zielbohrungen aufweist, die fluchtend zu zusätzlichen Querbohrungen in dem implantierten oder in einem anderen zugeordneten Frakturnagel ausrichtbar sind. Das Zielgerät muß dann nicht von einem implantierten Frakturnagel abgenommen werden. Man erreicht in Verbindung mit den erfindungsgemäß für sich schon vorteilhaften Ziel-Verstellpositionen eine weitere Verbesserung operativer Technik und Möglichkeiten.

Zweckmäßig kann ein einen verstellbaren Zielkopf lagernder unverstellbarer Teil eines Zielkopfes z.B. mit lösbaren Befestigungslaschen an dem Zielarm angebracht sein.

Hinsichtlich guter Griffigkeit und Bedienbarkeit zur Verstellung kann man den Zielkopf im wesentlichen olivenförmig oder kugelförmig ausbilden. Der Zielkopf weist dann einen Querschnitt und/oder Längsschnitt mit im wesentlichen ovalen oder kreisförmigen Konturen auf. Eine gerundete konvexe Außenfläche begünstigt auch das Einbringen von Zielhülsen bzw. Werkzeugen in die Zielbohrungen. Zudem sind dann, wenn der Zielkopf bei z.B. übergewichtigen Patienten an der Haut zur Anlage und in Weichteile gedrückt werden sollte, Verletzungen oder Irritationen vermieden. Dreh- und/oder Schiebeverstellung des Zielkopfes bleiben ohne Hautverletzung oder Beeinträchtigung gewährleistet.

Von besonderem Vorteil ist es, daß der erfindungsgemäße Zielkopf wenigstens zwei Zielbohrungen aufweisen kann, die auf unterschiedliche Durchgangsbereiche ein und desselben Durchgangsloches eines Frakturnagels ausrichtbar sind. Insbesondere kann so optional eine dynamische oder statische Positionierung einer Verriegelungsschraube vorgesehen werden.

Infolge der erfindungsgemäßen Verstell-Lagerung des Zielkopfes an dem freien Zielarmende mit den Zielpositionen zugeordneten Verstellwegen bzw. -winkeln erreicht man eine optimale Raumnutzung des Körpers des Zielkopfes zur Anordnung und Orientierung der Zielbohrungen. Zweckmäßig kann vorgesehen werden, daß wenigstens zwei Zielbohrungen windschief sind, also nicht in einer Ebene liegen, so daß sie weder parallel, noch sich schneidend verlaufen. Auch können Zielbohrungen vorteilhaft derart angeordnet werden, daß ein spitzer Winkel und/oder der Abstand zwischen wenigstens zwei Zielbohrungen möglichst groß sind. Auch können Zielbohrungen vorteilhaft derart angeordnet werden, daß sich möglichst wenige von ihnen kreuzen und/oder überlappen. Gegebenenfalls kann dadurch die Stabilität des Zielkopfes und/oder die Fläche der Bohrungen zum genauen Halten von Zielhülsen, Werkzeugen od.dgl. erhöht werden. Andernfalls könnten gegebenenfalls bei sich kreuzenden und/oder überlappenden Zielbohrungen Wandstärken zum Kreuzungsbereich hin abnehmen, so daß die Fläche umlaufender, an der Zielhülse bzw. dem Werkzeug anliegender und stützender Fläche reduziert wäre.

Innerhalb wenigstens einer Zielbohrung kann ein Selbsthemmungsmittel zum Festsetzen einer entfernbar in die Zielbohrung einsetzbaren Zielhülse angeordnet sein. Auch eine solche Gestaltung wird dadurch begünstigt, daß es möglich ist, die Zielbohrungen jedenfalls in Teilbereichen des Zielkopfkörpers mit relativ großem Wandabstand auszuführen. Insbesondere kann in Ausgestaltungen vorgesehen werden, daß das Selbsthemmungsmittel ein in der Zielbohrung nach innen hervorstehendes plastisch oder elastisch verformbares Druckelement umfaßt. Das Druckelement kann ein Ring sein, der in einer Umfangsnut der Zielbohrung sitzt. Es kann auch eine Manschette sein, die wenigstens einen Teil der Zielbohrung bedeckt.

Unteransprüche sind auf die genannten und noch andere zweckmäßige und vorteilhafte Ausgestaltungen der Erfindung gerichtet. Besonders zweckmäßige und vorteilhafte Ausbildungsformen oder -möglichkeiten der Erfindung werden anhand der folgenden Beschreibung der in der schematischen Zeichnung dargestellten Ausführungsbeispiele näher beschrieben. Es zeigen
- Fig. 1: in Seitenansicht von rechts ein erfindungsgemäßes Zielgerät für einen Femurnagel,
- Fig. 2: in perspektivischer Ansicht von rechts vom das Zielgerät der Fig. 1,
- Fig.3: in perspektivischer Ansicht von links hinten und in Explosionsdarstellung das Zielgerät der Fig. 1,
- Fig. 4: in perspektivischer Ansicht von links vom unten und in Explosionsdarstellung das Zielgerät der Fig. 1,
- Fig. 5: in perspektivischer Ansicht von links vom oben und in Explosionsdarstellung das Zielgerät der Fig. 1,
- Fig. 6: einen vergrößerten Querschnitt durch das freie Zielarmende und den Zielkopf des Zielgerätes der Fig. 1, und
- Fig. 7: in perspektivischer Ansicht von rechts vom das Zielgerät der Fig. 1, das mit einem Femurnagel verbunden ist und einen Bohrer führt.

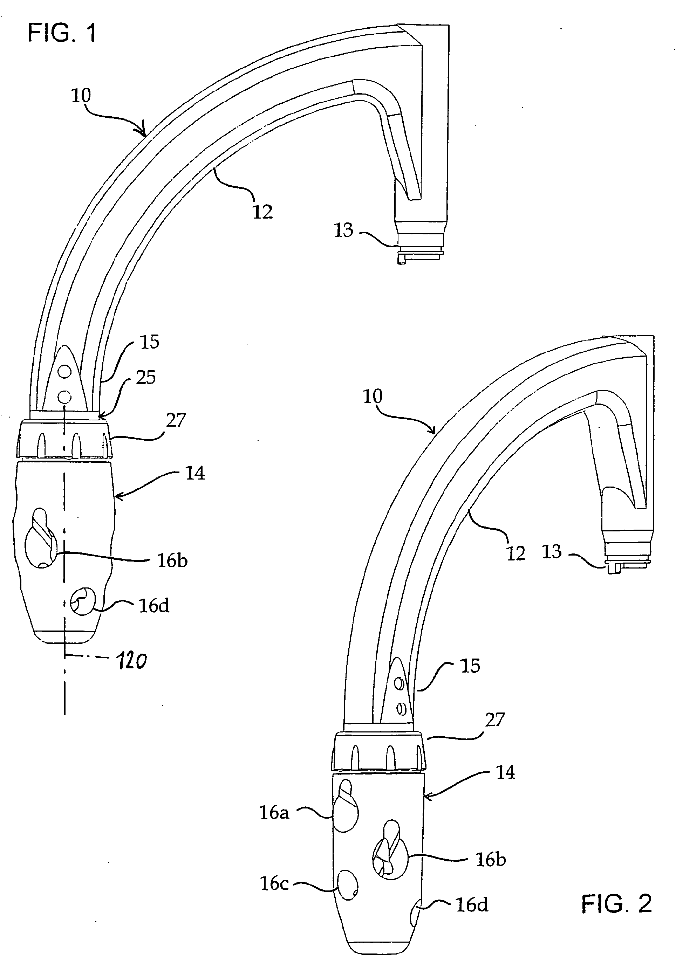

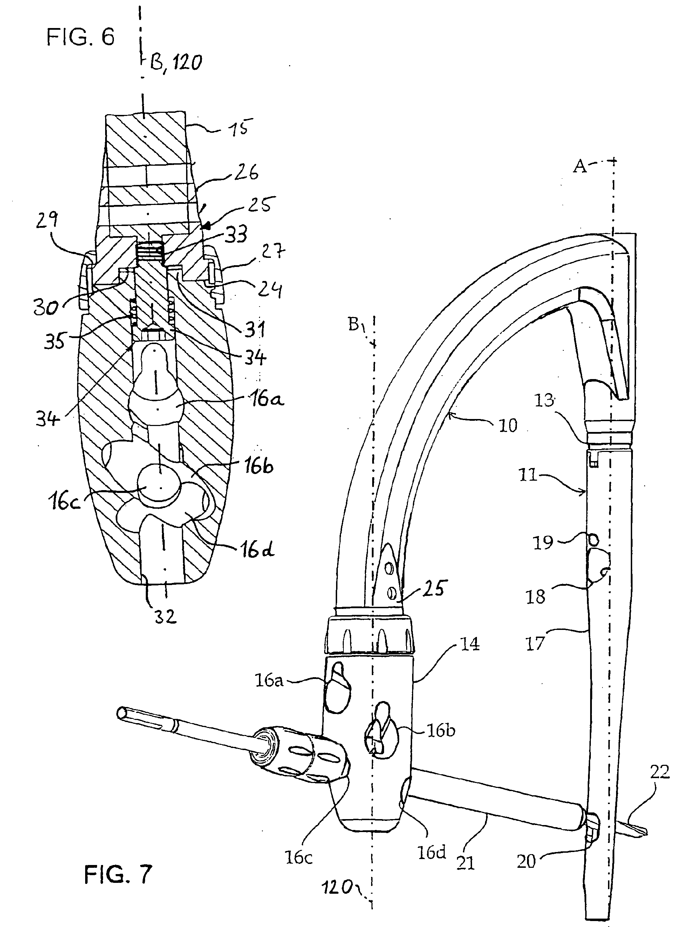

In Fig. 1 bis 7 ist ein Zielgerät 10 in bevorzugter Ausführungsform für einen Frakturnagel in Form eines üblichen Femurnagels 11 (vgl. Fig. 7) dargestellt. Die lateral zu liegen kommende Seite des Zielgeräts 10 wird als Vorderseite des Geräts betrachtet.

Das Zielgerät 10 umfaßt einen gebogenen Zielarm 12, der an seinem hinteren Ende Verbindungsmittel 13 für den Femurnagel 11 aufweist, und einen Zielkopf 14, der an dem anderen, vorderen freien Ende 15 des Zielarmes 12 abnehmbar angebracht ist und vier Zielbohrungen 16a, 16b, 16c, 16d aufweist.

Gemäß Fig. 7 umfaßt der Femurnagel 11 einen Marknagel (Nagelschaft) 17 sowie eine Schenkelhalsschraube (nicht dargestellt), einen dünneren Schenkelhalsstift (nicht dargestellt) und eine Knochenschraube (nicht dargestellt). Der Marknagel 17 weist drei quer zu seiner Längsachse A verlaufende, in einer gemeinsamen Zielebene liegende Bohrungen 18, 19, 20 auf, nämlich in seinem proximalen Bereich eine große erste Schrägbohrung 18 zur Aufnahme der Schenkelhalsschraube sowie in geringem Abstand darüber eine kleine parallele zweite Schrägbohrung 19 zur Aufnahme des Schenkelhalsstiftes und in seinem distalen Bereich ein Langloch 20 zur Aufnahme der Knochenschraube. Die beiden Schrägbohrungen 18, 19 verlaufen in einem Winkel von 130° zu der Längsachse A des Marknagels 17.

Die Verbindungsmittel 13 umfassen eine nach unten vorspringende Nase, die genau in eine Ausnehmung in der proximalen Stirnfläche des Femurnagels 11 paßt, wodurch eine Relativdrehung zwischen Zielgerät 10 und Femurnagel 11 verhindert wird.

Der Zielkopf 14 ist um eine Drehachse B, die parallel zu der Längsachse A und in der Zielebene liegt, drehbar, so daß jede der vier Zielbohrungen 16a bis 16d durch Drehen des Zielkopfes 14 fluchtend zu der ihr zugeordneten Querbohrung 18, 19, 20 ausgerichtet werden kann, wie im folgenden näher erläutert wird. Die Drehachse B verläuft koaxial mit einer Achse 120, die sich in Verlängerung des freien Zielarmendes 15 erstreckt und auch als Längsachse des freien Zielarmendes 15 bezeichnet werden kann.

Die erste Zielbohrung 16a fluchtet in einer ersten Drehstellung mit den beiden Schrägbohrungen 18, 19, verläuft also im gleichen Winkel von 130° zur Drehachse B. Die zweite Zielbohrung 16b verläuft hingegen in einem anderen Winkel von 125° zur Drehachse B und windschief zur ersten Zielbohrung 16a. Das heißt, daß die beiden Bohrungen 16a und 16b nicht in einer Ebene liegen, also weder parallel sind, noch sich schneiden. Die Zielbohrung 16b ist für einen anderen Femurnagel (nicht dargestellt) gedacht, dessen erste und zweite Schrägbohrung in gleichen Winkeln von 125° zu der Längsachse seines Marknagels verlaufen. Wenn dieser andere Femurnagel an Stelle des Femurnagels 11 der Fig. 7 mit dem Zielgeräts 10 verbunden ist, dann kann der Zielkopf 14 in eine zweite Drehstellung verstellt werden, in der die zweite Zielbohrung 16b mit den entsprechenden anderen beiden Schrägbohrungen fluchtet.

Die dritte Zielbohrung 16c fluchtet in einer dritten Drehstellung, die in Fig. 7 dargestellt ist, mit dem oberen Ende des Langloches 20. Die vierte Zielbohrung 16d verläuft windschief zu der dritten Zielbohrung 16c, so daß sie in einer vierten Drehstellung mit der Mitte des Langloches 20 fluchtet, wobei sie in dem gleichen Winkel zur Längsachse A wie die dritte Zielbohrung 16c in der dritten Drehstellung verläuft. Mit der vierten Zielbohrung 16d wird eine Verriegelungsschraube gesetzt, die proximal und distal von den Wänden des Langlochs 20 frei bleibt, so daß die Verriegelungsschraube dynamisch positioniert wird.

In Fig. 7 ist eine Zielhülse 21 dargestellt, die in der dritten Zielbohrung 16c geführt ist und ihrerseits einen Bohrer 22 führt, der durch das obere Ende bzw. den proximalen Bereich des Langloches 20 hindurchgeht, um ein Bohrloch für die zu setzende Knochenschraube zu schaffen.

In dieses Bohrloch, das durch Weichteilgewebe und vor und hinter dem Langloch 20 liegende Knochenbereiche hindurchgeht und im Weichteilgewebe durch die Zielhülse 21 offen gehalten wird, wird, nachdem der Bohrer 22 aus der in der Zielbohrung 16c verbleibenden Zielhülse 21 entnommen worden ist, die Knochenschraube durch die nun freie Zielhülse 21 und das Langloch 20 bis zum Anschlag am Boden des Bohrloches im medial (hinten) liegenden Knochen geschoben. Anschließend wird sie weiter in den Knochen hineingeschraubt, so daß sie den Marknagel 17 im Femur verriegelt. Schließlich wird die Zielhülse 21 aus dem Weichteilgewebe und der Zielbohrung 16c herausgezogen. Die Knochenschraube kommt gegen die proximale Wand des Langloches 20 zur Anlage, so daß eine statische Verriegelung hergestellt wird.

Der Durchmesser der Zielbohrung 16c ist größer als die Breite des Langloches 20, da sie ja die Zielhülse 21 aufnehmen muß, deren Innendurchmesser der Langlochbreite und dem Durchmesser der Knochenschraube entspricht.

Die Zielhülse 21 dient als Verlängerung der Zielbohrung 16c und sorgt somit für eine genaue Führung des Bohrers 22, der frei drehbar und daher nicht genau passend, sondern mit etwas Radialspiel in der Zielhülse 21 sitzen muß. Die Zielhülse 21 soll jedoch sicher und präzise, also ohne zu wackeln, in der Zielbohrung 16c sitzen. Zu diesem Zweck ist in einer Umfangsnut der Zielbohrung 16c als Selbsthemmungsmittel ein nicht dargestellter elastisch verformbarer Gummiring gehalten, der nach innen vorsteht und somit die Zielhülse 21 festklemmt.

Nach dem Entfernen der Zielhülse 21 kann beispielsweise der Zielkopf 14 in die erste Drehstellung verstellt werden, um die Schenkelhalsschraube und den Schenkelhalsstift mit Hilfe der ersten Zielbohrung 16a in die beiden Schrägbohrungen 18, 19 einzuführen. Zu diesem Zweck wird in einem ersten Schritt eine Doppel-Zielhülse (nicht dargestellt) in die Zielbohrung 16a eingeführt. Diese Doppel-Zielhülse weist im Unterschied zu der Einfach-Zielhülse 21 nicht einen, sondern zwei Kanäle auf, die im Abstand der beiden Schrägbohrungen 18, 19 parallel zueinander verlaufen und deren Innendurchmesser den Innnendurchmessern der jeweils zugeordneten Schrägbohrungen 18, 19 entsprechen. Die Kontur der Doppel-Zielhülse entspricht dem Querschnitt der Zielbohrung 16a, der durch einen großen und einen kleinen Kreis gebildet wird, die in Schlüsselloch ähnlicher Form miteinander verschmolzen sind und in der ersten Drehstellung koaxial mit den beiden Schrägbohrungen 18, 19 sind. In der Zielbohrung 16a kann daher auch eine Einfach-Zielhülse mit einer Kontur entsprechend dem großen Kreis sicher gehalten werden.

In einem zweiten Schritt wird die Doppel-Zielhülse durch das Weichteilgewebe bis zum Femur geschoben. In einem dritten Schritt wird zunächst ein Bohrer (nicht dargestellt) in den großen Kanal eingeführt, dann wird ein mit der Schrägbohrung 18 fluchtendes Bohrloch für die zu setzende Schenkelhalsschraube durch den Knochen gebohrt, und schließlich wird der Bohrer aus der Doppel-Zielhülse herausgezogen. In einem vierten Schritt wird die Schenkelhalsschraube durch den großen Kanal in die Schrägbohrung 18 und das Bohrloch eingeführt und in den Schenkelhalskopf geschraubt.

Der zu setzende Schenkelhalsstift wird auf die gleiche Art unter Verwendung eines dünneren Bohrers und des dünnen Kanals in die Schrägbohrung 19 eingeführt und im Schenkelhals parallel zur Schenkelhalsschraube verankert.

Anstelle der ersten und zweiten Schlüsselloch-förmigen Zielbohrung 16a, 16b kann der Zielkopf 14 jeweils zwei den zugeordneten parallelen Querbohrungen entsprechende parallele Zielbohrungen aufweisen, die fluchtend mit den zugeordneten Querbohrungen ausgerichtet sind oder werden. Erfindungsgemäß können zweckmäßig relativ zur Zielebene verstellbare Zielbohrungen z.B. auch für die Querbohrungen 18, 19 ausgebildet werden, die dann unter spitzem Winkel sowie nicht in gemeinsamer Ebene in dem Zielkopf 14 verlaufen. Verstell- und Zielbohrungsanordnung entsprechen dann der für die Zielbohrungen 16c und 16d.

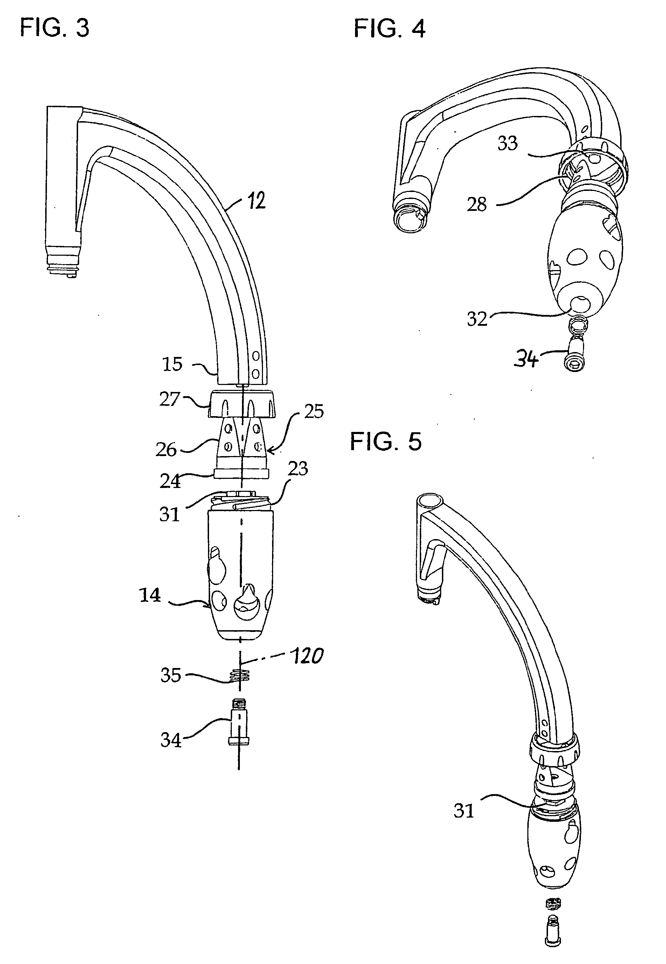

Im folgenden werden anhand der Fig. 3 bis 6 Aufbau und Funktionsweise des Zielkopfes 14 näher erläutert.

Der Zielkopf 14 hat eine im wesentlichen olivenförmige, längs der Drehachse B symmetrische und sich langerstreckende Grundform und weist an seinem oberen, zum freien Zielarmende 1 weisenden Ende ein Außengewinde 23 auf. Das freie Zielarmende 15 weist an seinem unteren, zum Zielkopf 14 weisenden Ende einen radial nach außen vorspringenden Außenflansch 24 auf. Der Außenflansch 24 ist am unteren Rand einer Muffe 25 ausgebildet, die zwei nach oben ragende Befestigungsflügel oder Laschen 26 aufweist. Die Flügel 26 liegen an gegenüberliegenden Seiten des Zielarmendes 15 an und sind an diesem mit Hilfe von zwei nicht dargestellten Schrauben befestigt.

Auf der Muffe 25 sitzt eine Überwurfmutter 27, die an ihrem unteren Rand ein zu dem Außengewinde 23 passendes Innengewinde 28 und an ihrem oberen Rand einen zu dem Außenflansch 24 passenden, radial nach innen vorspringenden Innenflansch 29 aufweist. Der Innenflansch 29 ist derart ausgebildet, daß er, wenn die Überwurfmutter 27 auf dem Zielkopf 14 angezogen wird, mit einer unteren, zum Zielkopf 14 weisenden Stirnfläche an der oberen, vom Zielkopf 14 abgewandten Stirnfläche des Außenflansches 24 anliegt. Die Überwurfmutter 27 bildet ein Spann- und Klemmittel, mit dem der Zielarm 12 und der Zielkopf 14 in gegeneinander eingestellter Verstellposition gegen Verstellung aus der eingestellten Rastposition gesichert sind. Zudem hält die Überwurfmutter 27 das Zielarmende 15 und den Zielkopf 14 in unverlierbarem Steckeingriff. Man erkennt, daß die Muffe 25 ein unverstellbares Teil eines die Teile 25, 27 sowie den drehverstellbaren Zielkopf 14 umfassenden Zielkopfes ist, der durch Lösen der Laschenverbindung von dem Zielarmende 15 abnehmbar ist.

Zum Verstellen des Zielkopfes 14 wird die Überwurfmutter 27 losgeschraubt, so daß er relativ zum Zielarm 12 in die gewünschte Drehstellung gedreht werden kann.

Zwischen dem Zielarmende 15 und dem Zielkopf 14 ist ein besonderes Feststellmittel angebracht, das durch ein Rastmittel zur stufigen Rastverstellung der Dreh-Zielpositionen des Zielkopfes 14 gebildet ist. Das freie Zielarmende 15 weist eine Rastvertiefung 30 auf, die in der unteren Stirnfläche der Muffe 25 ausgebildet ist. Der Grundriß der Rastvertiefung 30 ist ein regelmäßiges Vieleck, und zwar im Ausführungsbeispiel ein Sechseck, das mit seiner zentralen Hauptachse koaxial zu der Drehachse B liegt. Der Zielkopf 14 hingegen weist an seiner oberen Stirnfläche einen Rastvorsprung 31 auf, dessen Grundriß dem Grundriß der Rastvertiefung 30 gleicht und somit ebenfalls ein regelmäßiges Sechseck ist. Der Zielkopf 14 kann also nur sechs verschiedene, in gleichen Dreh-Winkelabständen vorgegebene Drehstellungen einnehmen, in denen der Rastvorsprung 31 in der Rastvertiefung 30 sitzt. Vier von diesen Drehstellungen korrespondieren mit den oben beschriebenen Drehstellungen.

Der Zielkopf 14 ist zudem zu dem freien Zielarmende 15 hin vorgespannt, so daß auch bei losgedrehter Überwurfmutter 27 der Rastvorsprung 31 in der Rastvertiefung 30 in Steckverbindung sitzen bleibt. Zu diesem Zweck weist der Zielkopf 14 eine Längsbohrung 32 auf, die ausgehend von dessen unterer Stirnfläche koaxial mit der Drehachse B bis zu dessen oberer Stirnfläche verläuft. Außerdem weist das freie Zielarmende 15 in seiner Stirnfläche eine Sackbohrung 33 mit Innengewinde auf. Schließlich ist eine Schraube 34, auf deren Schaft eine Schraubenfeder 35 sitzt, von unten in die Längsbohrung 32 eingeführt und in die Sackbohrung 33 hineingeschraubt. Da die Schraubenfeder 35 sich mit ihrem oberen Ende an einer Anschlagfläche in der Längsbohrung 32 und mit ihrem unteren Ende an dem Kopf der Schraube 34 abstützt, hält sie den Zielkopf 14 gegen das Zielarmende 15 gedrückt.

Zum Verstellen des Zielkopfes 14 wird er bei losgeschraubter Überwurfmutter 27 relativ zum Zielarmende 15 gegen die Vorspannkraft der Schraubenfeder 35 soweit nach unten gezogen, bis der Rastvorsprung 31 nicht mehr in der Rastvertiefung 30 sitzt. Zum Abnehmen des Zielkopfes 14 vom Zielarm 12 wird die Schraube 34 aus der Sackbohrung 33 herausgeschraubt und die Überwurfmutter 27 von dem Zielkopf 15 abgeschraubt.

## Patentansprüche

1. Zielgerät (10) für einen Frakturnagel (11), umfassend einen Zielarm (12), der an seinem einen Ende ein Verbindungsmittel (13) zur lösbaren Verbindung mit dem Frakturnagel (11) aufweist, wenigstens einen an dem anderen freien Ende (15) des Zielarms (12) angeordneten verstellbaren Aufnahmeabschnitt mit Zielbohrung (16a-16d), die einer quer zur Nagel-Längsachse (a) verlaufenden Frakturnagel-Querdurchbohrung (18-20) zur Aufnahme eines Knochenstiftes zugeordnet ist, sowie ein Feststellmittel zum Feststellen des Aufnahmeabschnitts an dem freien Zielarmende (15), **dadurch gekennzeichnet, daß** der Aufnahmeabschnitt durch einen Zielkopf (14) gebildet ist, der zum Einstellen in unterschiedliche Zielpositionen für ein und denselben Frakturnagel (11) stufig verstellbar ist, und daß das Feststellmittel durch ein Rastmittel (30-35) zur stufigen Verstellung des Zielkopfes (14) in wenigstens zwei vorgegebene Rastpositionen gebildet ist, wobei für ein und denselben Frakturnagel (11) jede Rastposition eine Zielposition für eine zugeordnete Frakturnagel-Querdurchbohrung (18-20) bestimmt.

2. Zielgerät nach Anspruch 1, **dadurch gekennzeichnet, daß** das Rastmittel (30-35) Mittel (30, 31) zur stufigen Drehverstellung umfaßt.

3. Zielgerät nach Anspruch 2, **dadurch gekennzeichnet, daß** der Zielkopf (14) zur stufigen Drehverstellung in die Rastpositionen bestimmenden Winkelabständen um eine Drehachse (B) drehverstellbar ist.

4. Zielgerät nach Anspruch 2 oder 3, **dadurch gekennzeichnet, daß** der Zielkopf (14) um eine Achse (B,120) drehbar ist, die sich in Verlängerung des freien Zielarmendes (15) erstreckt.

5. Zielgerät nach einem der Ansprüche 2 bis 4, **dadurch gekennzeichnet, daß** der Zielkopf (14) um eine Achse (B) drehbar ist, die in gemeinsamer Ebene mit der Längsachse (A) eines mit dem Zielgerät (10) verbundenen Frakturnagels (11) zu liegen kommt.

6. Zielgerät nach einem der Ansprüche 2 bis 5, **dadurch gekennzeichnet, daß** der Zielkopf (14) um eine Achse (B) drehbar ist, die zumindest im wesentlichen parallel zur Längsachse eines mit dem Zielgerät (10) verbundenen Frakturnagels (11) liegt.

7. Zielgerät nach einem der Ansprüche 2 bis 6, **dadurch gekennzeichnet, daß** der Zielkopf um eine Achse drehbar ist, die zumindest im wesentlichen rechtwinklig zu der Ebene liegt, die von der Längsachse eines mit dem Zielgerät verbundenen Frakturnagels und einer in Verlängerung des freien Zielarmendes sich erstreckenden Achse aufgespannt wird.

8. Zielgerät nach einem der Ansprüche 2 bis 7, **dadurch gekennzeichnet, daß** der Zielkopf um eine Achse drehbar ist, die zumindest im wesentlichen rechtwinklig zu der Längsachse eines mit dem Zielgerät verbundenen Frakturnagels und in der Ebene liegt, die von der Längsachse des Frakturnagels und einer in Verlängerung des freien Zielarmendes sich erstreckenden Achse aufgespannt wird.

9. Zielgerät nach einem der Ansprüche 1 bis 8, **dadurch gekennzeichnet, daß** der Zielkopf (14) verschiebbar, vorzugsweise in Richtung einer in Verlängerung des feien Zielarmendes (15) sich erstreckender Achse (120), an dem freien Zielarmende (15) angebracht ist.

10. Zielgerät nach einem der Ansprüche 1 bis 9, **dadurch gekennzeichnet, daß** das Rastmittel (30-35) ein zwischen dem freien Zielarmende (15) und dem Zielkopf (14) ausgebildetes Spann- oder Klemmittel (27) ist, mit dem Zielarm (12) und Zielkopf (14) in gegeneinander eingestellter Verstellposition gegen Verstellung gesichert werden.

11. Zielgerät nach einem der Ansprüche 1 bis 10, **dadurch gekennzeichnet, daß** das Rastmittel (30-35) ein den Zielkopf (14) gegen das freie Zielarmende (15) vorspannendes Federmittel (35) umfaßt, mittels dessen der Zielkopf (14) unter auf ihn gegen die Vorspannrichtung ausgeübter Zugkraft zwischen den Rastpositionen drehverstellbar ist.

12. Zielgerät nach einem der Ansprüche 1 bis 11, **dadurch gekennzeichnet, daß** der Zielkopf (14) lösbar an dem freien Zielarmende (15) angebracht ist.

13. Zielgerät nach Anspruch 12, **dadurch gekennzeichnet, daß** der Zielkopf (14) mittels Schraubverbindung und/oder Steckverbindung (27, 33, 34; 30, 31) mit dem freien Zielarmende (15) verbunden ist.

14. Zielgerät nach Anspruch 12 oder 13, **dadurch gekennzeichnet, daß** ein unverstellbarer Teil (25) eines den verstellbaren Zielkopf (14) aufweisenden Zielkopfes an dem freien Zielarmende (15) befestigt ist.

15. Zielgerät nach einem der Ansprüche 13 oder 14, **dadurch gekennzeichnet, daß** die Schraubverbindung eine durch den Zielkopf (14) hindurchgehende, Zielbohrungen (16a-16d) kreuzende Längsbohrung (32) und eine durch die Längsbohrung (32) in den Zielkopf (14) einbringbare Schraube (34) umfaßt, die in mit dem freien Zielarmende (15) verschraubter Position sämtliche Zielbohrungen (16a-16d) frei läßt.

16. Zielgerät nach einem der Ansprüche 13 bis 15, **dadurch gekennzeichnet, daß** die Schraubverbindung eine an dem freien Zielarmende (15) gelagerte Überwurfmutter (27) umfaßt, mittels der das Zielarmende (15) und der Zielkopf (14) in unverlierbarem Eingriff gehalten werden.

17. Zielgerät nach einem der Ansprüche 1 bis 16, **dadurch gekennzeichnet, daß** der Zielkopf (14) im wesentlichen olivenförmig oder kugelförmig ist.

18. Zielgerät nach einem der Ansprüche 1 bis 17, **dadurch gekennzeichnet, daß** der Zielkopf (14) wenigstens zwei Zielbohrungen (16c, 16d) aufweist, die in Zielpositionen auf unterschiedliche Durchgangsbereiche ein und desselben Durchgangsloches (20) eines Frakturnagels (11) ausrichtbar sind.

19. Zielgerät nach einem der Ansprüche 1 bis 18, **dadurch gekennzeichnet, daß** wenigstens zwei Zielbohrungen (16a, 16b) nicht in einer Ebene liegen, so daß sie weder parallel, noch sich schneidend verlaufen.

20. Zielgerät nach einem der Ansprüche 1 bis 19, **dadurch gekennzeichnet, daß** Zielbohrungen (16a-16d) derart angeordnet sind, daß spitzer Winkel und/oder Abstand zwischen wenigstens zwei Zielbohrungen (16a, 16d) möglichst groß sind.

## Claims

1. Targeting device (10) for a fracture nail (11), comprising a targeting arm (12) having at one end thereof a connecting means (13) for releasable connection with the fracture nail (11), at least one adjustable receiving section with targeting bore (16a-16d) arranged at the other free end (15) of the targeting arm (12), the targeting bore (16a-16d) being associated with a fracture nail cross through-bore (18-20) extending transversely to the longitudinal axis (a) of the nail for receiving a bone pin, and a fastening means for fastening the receiving section at the free end (15) of the targeting arm, **characterized in that** the receiving section is formed by a targeting head (14) being adjustable in steps for adjustment in different targeting positions for one and the same fracture nail (11), and that the fastening means is formed by a latch means (30-35) for adjusting the targeting head (14) in steps in at least two predetermined latching positions, each latch position determining a targeting position for an associated fracture nail cross through-bore (18-20) for one and the same fracture nail (11).

2. Targeting device according to claim 1, **characterized in that** the latch means (30-35) comprises means (30, 31) for stepwise rotational adjustment.

3. Targeting device according to claim 2, **characterized in that** the targeting head (14) is rotationally adjustable around a rotational axis (B) for stepwise rotational adjustment in angular distances determining the latching positions.

4. Targeting device according to claim 2 or 3, **characterized in that** the targeting head (14) is rotatable around an axis (B,120) extending in projection of the free end (15) of the targeting arm.

5. Targeting device according to any one of claims 2 to 4, **characterized in that** the targeting head (14) is rotatable around an axis (B) which comes to lie in a common plane with the longitudinal axis (A) of a fracture nail (11) connected with the targeting device (10).

6. Targeting device according to any one of claims 2 to 5, **characterized in that** the targeting head (14) is rotatable around an axis (B) which lies at least essentially parallel to the longitudinal axis of a facture nail (11) connected with the targeting device (10).

7. Targeting device according to any one of claims 2 to 6, **characterized in that** the targeting head is rotatable around an axis which lies at least essentially at right angles to the plane being spanned by the longitudinal axis of a fracture nail connected with the targeting device and an axis extending in projection of the free end of the targeting arm.

8. Targeting device according to any one of claims 2 to 7, **characterized in that** the targeting head is rotatable around an axis which lies at least essentially at right angles to the longitudinal axis of a fracture nail connected with the targeting device and in the plane being spanned by the longitudinal axis of the fracture nail and an axis extending in projection of the free end of the targeting arm.

9. Targeting device according to any one of claims 1 to 8, **characterized in that** the targeting head (14) is mounted displaceably, preferably in the direction of an axis (120) extending in projection of the free end (15) of the targeting arm, on the free end (15) of the targeting arm.

10. Targeting device according to any one of claims 1 to 9, **characterized in that** the latch means (30-35) is a spanning or clamping means (27) formed between the free end (15) of the targeting arm and the targeting head (14), by means of which targeting arm (12) and targeting head (14) are secured against displacement in a mutually set adjustment position.

11. Targeting device according to any one of claims 1 to 10, **characterized in that** the latch means (30-35) comprises a spring means (35) biasing the targeting head (14) against the free end (15) of the targeting arm, by means of which the targeting head (14) is rotatably adjustable between the latching positions under tensile force exerted on it against the biasing direction.

12. Targeting device according to any one of claims 1 to 11, **characterized in that** the targeting head (14) is releasably mounted on the free end (15) of the targeting arm.

13. Targeting device according to claim 12, **characterized in that** the targeting head (14) is connected to the free end (15) of the targeting arm by means of screw coupling and/or plug connection (27, 33, 34; 30, 31).

14. Targeting device according to claim 12 or 13, **characterized in that** a non-adjustable part (25) of a targeting head comprising the adjustable targeting head (14) is mounted on the free end (15) of the targeting arm.

15. Targeting device according to claim 13 or 14, **characterized in that** the screw coupling comprises a longitudinal bore (32) extending through the targeting head (14) and crossing targeting bores (16a-16d) and a screw (34) which can be inserted into the targeting head (14) through the longitudinal bore (32), wherein the screw (34) leaves all targeting bores (16a-16d) open in a position screwed together with the free end (15) of the targeting arm.

16. Targeting device according to any one of claims 13 to 15, **characterized in that** the screw coupling comprises a connecting nut (27) mounted on the free end (15) of the targeting arm, by means of which the end (15) of the targeting arm and the targeting head (14) are held in undetachable engagement.

17. Targeting device according to any one of claims 1 to 16, **characterized in that** the targeting head (14) is essentially olive-shaped or spherical.

18. Targeting device according to any one of claims 1 to 17, **characterized in that** the targeting head (14) comprises at least two targeting bores (16c, 16d) which may be directed towards different passing regions of one and the same through-hole (20) of a fracture nail (11) in targeting positions.

19. Targeting device according to any one of claims 1 to 18, **characterized in that** at least two targeting bores (16a, 16b) do not lie in one plane so that they extend neither in parallel nor intersectingly.

20. Targeting device according to any one of claims 1 to 19, **characterized in that** targeting bores (16a-16d) are arranged in such a way that an acute angle and/or a distance between at least two targeting bores (16a, 16d) are as large as possible.

## Revendications

1. Dispositif de visée (10) pour une broche à fracture (11), comportant un bras de visée (12) comprenant sur une de ses extrémités des moyens de liaison (13) destinés à le relier de manière amovible à la broche à fracture (11), au moins une partie de réception mobile disposée sur l'autre extrémité libre (15) du bras de visée (12) et dotée d'un alésage de visée (16a-16d) associé à un alésage latéral de broche à fracture (18-20) destiné à recevoir un clou à os et s'étendant transversalement à l'axe longitudinal (a) de la broche, ainsi que des moyens de blocage destinés à bloquer la partie de réception sur l'extrémité libre (15) du bras de visée, **caractérisé en ce que** la partie de réception est formée par une tête de visée (14) pouvant être déplacée graduellement pour le réglage dans différentes positions de visée pour une seule et même broche à fracture (11), et **en ce que** les moyens de blocage sont formés par des moyens d'enclenchement (30-35) destinés à déplacer graduellement la tête de visée (14) dans au moins deux positions d'enclenchement prédéterminées, chaque position d'enclenchement déterminant une position de visée pour un alésage latéral de broche à fracture (18-20) associé pour une seule et même broche à fracture (11).

2. Dispositif de visée selon la revendication 1, **caractérisé en ce que** les moyens d'enclenchement (30-35) comportent des moyens (30, 31) destinés au déplacement rotatif graduel.

3. Dispositif de visée selon la revendication 2, **caractérisé en ce que** la tête de visée (14) destinée au déplacement rotatif graduel peut être déplacée de manière rotative autour d'un axe de rotation (B) suivant des espacements angulaires déterminant les positions d'enclenchement.

4. Dispositif de visée selon la revendication 2 ou 3, **caractérisé en ce que** la tête de visée (14) peut tourner autour d'un axe (B, 120) s'étendant dans le prolongement de l'extrémité libre (15) du bras de visée.

5. Dispositif de visée selon l'une quelconque des revendications 2 à 4, **caractérisé en ce que** la tête de visée (14) peut tourner autour d'un axe (B) venant se placer dans un plan commun avec l'axe longitudinal (A) d'une broche à fracture (11) reliée au dispositif de visée (10).

6. Dispositif de visée selon l'une quelconque des revendications 2 à 5, **caractérisé en ce que** la tête de visée (14) peut tourner autour d'un axe (B) au moins sensiblement parallèle à l'axe longitudinal d'une broche à fracture (11) reliée au dispositif de visée (10).

7. Dispositif de visée selon l'une quelconque des revendications 2 à 6, **caractérisé en ce que** la tête de visée peut tourner autour d'un axe au moins sensiblement perpendiculaire au plan déterminé par l'axe longitudinal d'une broche à fracture reliée au dispositif de visée et un axe s'étendant dans le prolongement de l'extrémité libre du bras de visée.

8. Dispositif de visée selon l'une quelconque des revendications 2 à 7, **caractérisé en ce que** la tête de visée peut tourner autour d'un axe au moins sensiblement perpendiculaire à l'axe longitudinal d'une broche à fracture reliée au dispositif de visée et situé dans le plan déterminé par l'axe longitudinal de la broche à fracture et un axe s'étendant dans le prolongement de l'extrémité libre du bras de visée.

9. Dispositif de visée selon l'une quelconque des revendications 1 à 8, **caractérisé en ce que** la tête de visée (14) est installée de manière mobile sur l'extrémité libre (15) du bras de visée, de préférence en direction d'un axe (120) s'étendant dans le prolongement de l'extrémité libre (15) du bras de visée.

10. Dispositif de visée selon l'une quelconque des revendications 1 à 9, **caractérisé en ce que** les moyens d'enclenchement (30-35) sont des moyens de tension ou de serrage (27) conçus entre l'extrémité libre (15) du bras de visée et la tête de visée (14) et permettant d'empêcher le bras de visée (12) et la tête de visée (14) de se déplacer lorsqu'ils se trouvent dans une position de déplacement mutuellement réglée.

11. Dispositif de visée selon l'une quelconque des revendications 1 à 10, **caractérisé en ce que** les moyens de blocage (30-35) comportent des moyens formant ressort (35) précontraignant la tête de visée (14) contre l'extrémité libre (15) du bras de visée et au moyen desquels la tête de visée (14) peut être déplacée en rotation entre les positions d'enclenchement sous l'effet d'une force de traction exercée sur elle dans le sens contraire de la précontrainte.

12. Dispositif de visée selon l'une quelconque des revendications 1 à 11, **caractérisé en ce que** la tête de visée (14) est installée de manière amovible sur l'extrémité libre (15) du bras de visée.

13. Dispositif de visée selon la revendication 12, **caractérisé en ce que** la tête de visée (14) est reliée à l'extrémité libre (15) du bras de visée au moyen d'un assemblage vissé et/ou d'un assemblage par emboîtement (27, 33, 34 ; 30, 31).

14. Dispositif de visée selon la revendication 12 ou 13, **caractérisé en ce qu'**une partie immobile (25) d'une tête de visée comprenant la tête de visée mobile (14) est fixée à l'extrémité libre (15) du bras de visée.

15. Dispositif de visée selon l'une quelconque des revendications 13 ou 14, **caractérisé en ce que** l'assemblage vissé comporte un alésage longitudinal (32) traversant la tête de visée (14) et croisant les alésages de visée (16a-16d) et une vis (34) pouvant être introduite à travers l'alésage longitudinal (32) dans la tête de visée (14) et laissant tous les alésages de visée (16a-16d) dégagés dans la position vissée avec l'extrémité libre (15) du bras de visée.

16. Dispositif de visée selon l'une quelconque des revendications 13 à 15, **caractérisé en ce que** l'assemblage vissé comporte un écrou-raccord (27) logé sur l'extrémité libre (15) du bras de visée et au moyen duquel l'extrémité libre (15) du bras de visée et la tête de visée (14) sont maintenues en prise indétachable.

17. Dispositif de visée selon l'une quelconque des revendications 1 à 16, **caractérisé en ce que** la tête de visée (14) est sensiblement en forme d'olive ou de sphère.

18. Dispositif de visée selon l'une quelconque des revendications 1 à 17, **caractérisé en ce que** la tête de visée (14) comprend au moins deux alésages de visée (16c, 16d) pouvant être orientés dans des positions de visée sur différentes zones de passage d'un seul et même trou de passage (20) d'une broche à fracture (11).

19. Dispositif de visée selon l'une quelconque des revendications 1 à 18, **caractérisé en ce qu'**au moins deux alésages de visée (16a, 16b) ne se situent pas dans un plan, de sorte qu'ils ne s'étendent ni parallèlement ni en se croisant.

20. Dispositif de visée selon l'une quelconque des revendications 1 à 19, **caractérisé en ce que** les alésages de visée (16a-16d) sont disposés de telle sorte que l'angle aigu et/ou l'espacement entre au moins deux alésages de visée (16a, 16d) soient les plus grands possible.
